# EUROPEAN PATENT APPLICATION

(11) **EP 3 791 888 A1**
(43) Date of publication of application: **17.03.2021**
(21) Application number: 19800676.9
(22) Date of filing: 09.05.2019
(51) Int. Cl.: A61K 35/28, A61P 7/00, A61P 25/00, C12N 5/0775

(54) **THERAPEUTIC AGENT FOR SPINAL CORD INJURY**

(30) Priority: 09.05.2018 JP 2018090979
(71) Applicant: Life Science Institute, Inc., Tokyo 100-8251 (JP)
(72) Inventor: KAWAMURA, Makoto, Tokyo 101-0047 (JP); MASUTOMI, Naoya, Tokyo 101-0047 (JP)
(74) Representative: Gill Jennings & Every LLP
(86) International application number: PCT/JP2019/018578
(87) International publication number: WO 2019/216380

(57) **Abstract**

A cell product for treatment of spinal cord injury, comprising a SSEA-3-positive pluripotent stem cell (Muse cell) derived from a mesenchymal tissue in a living body or a cultured mesenchymal cell. Preferably, the spinal cord injury is complete or incomplete spinal cord injury.

## Description

### TECHNICAL FIELD

The present invention relates to a cell product in regenerative therapy. More particularly, the present invention relates to a cell product comprising a pluripotent stem cell effective for treatment of spinal cord injury.

### BACKGROUND ART

Spinal cord injury is a condition in which a spinal cord is injured due to damage to the spine caused mainly by strong external forces applied to the spinal column. Similar disorders develop by internal causes, such as spinal cord tumors and hernias (hereafter referred to collectively as spinal injury).

It is said that Japan currently has more than 100,000 people with spinal injury, with more than 5,000 new patients with spinal cord injury occurring each year. The causes of injuries include, in descending order of percentage, 1. traffic accidents, 2. falls from high places, 3. falls, 4. bruises and crush under something, 5. sports, and 6. others.

Spinal cord injuries are divided into "complete type" and "incomplete type" according to the degree of injury. The "complete type" is referred to as "complete spinal cord injury," and is a condition in which a spinal cord is severed laterally and the neurotransmitter function is completely blocked. The "incomplete type" is referred to as "incomplete spinal cord injury," and is a condition in which a part of the spinal cord is subjected to trauma, compression, or the like with some function remaining.

It is said that, unlike peripheral nerves, the central nervous system, including the spinal cord, cannot be repaired or regenerated once it has been damaged.

Thus, as immediate treatments, secondary injuries to the spine or spinal cord are prevented, including immobilization, oxygenation, and maintenance of spinal cord perfusion, and supportive therapy. The acute phase high dose steroid therapy (Non-patent Document 1), which was reported for its effectiveness in animal experiments and clinical trials in the 1990s, is said to have an effect to reduce physical impediments. However, this therapy is said to be only effective in relatively young patients with strong regenerative capacity and be less effective in others, and thus is not general.

Thus, rehabilitation is usually carried out in the post-acute phase, as early as possible after injuries. However, rehabilitation for spinal cord injuries does not restore lost functions.

There is a demand for regenerating the destroyed spinal cord and recovering the function by patients with spinal injuries, for which researches are underway in a variety of fields. In particular, researches for stem cell-based regenerative therapy is being investigated for central nervous system diseases such as spinal cord injury, which were previously thought to be incurable.

For example, greatly various types of cells such as neural stem cells (Non-patent Document 2), embryonic stem cells (Non-patent Document 3), bone marrow mesenchymal stem cells (Non-patent Documents 4 and 5), and olfactory mucosal cells (Non-patent Document 6) have been used in animal experiments and reported to be effective in improving the pathology of spinal cord injury. Some are now being tested in clinical trials, but so far only autologous bone marrow mesenchymal stem cells (Non-patent Document 7) have been approved.

It has been found that pluripotent stem cells, which are present in mesenchymal cell fractions, can be obtained without gene introduction or induction by cytokines or the like, and express SSEA-3 (Stage-Specific Embryonic Antigen-3) as a surface antigen (Multilineage-differentiating Stress Enduring cells; Muse cell), can be responsible for the pluripotency possessed by the mesenchymal cell fractions, and applied to disease treatment aimed at tissue regeneration (e.g., Patent Document 1; Non-patent Documents 8 to 10). However, it has not been demonstrated whether Muse cells could provide expected therapeutic effects in treatment of spinal cord injury.

### PRIOR ART DOCUMENTS

### PATENT DOCUMENT

Patent Document 1: Japanese Patent No. 5185443

### NON-PATENT DOCUMENTS

Non-patent Document 1: Bracken MB, Shepard MJ, Collins WF, Holford TR, Young W, Baskin DS, Eisenberg HM, Flamm E, Leo-Summers L, Maroon: N Engl J Med. 322 :1405-1411, 1990.
Non-patent Document 2: Okada S, Ishii K, Yamane J, Iwanami A, Ikegami T, Katoh H, Iwamoto Y, Nakamura M, Miyoshi H, Okano HJ, Contag CH, Toyama Y, Okano H: FASEB J. 19: 1839-1841, 2005.
Non-patent Document 3: McDonald JW, Liu XZ, Qu Y, Liu S, Mickey SK, Turetsky D, Gottlieb DI, Choi DW: Nat Med. 5: 1410-1412, 1999.
Non-patent Document 4: Hofstetter CP, Schwarz EJ, Hess D, Widenfalk J, El Manira A, Prockop DJ, Olson L: Proc Natl Acad Sci U S A. 99 :2199-2204, 2002.
Non-patent Document 5: Koda M, Okada S, Nakayama T, Koshizuka S, Kamada T, Nishio Y, Someya Y, Yoshinaga K, Okawa A, Moriya H, Yamazaki M: Neuroreport.,16: 1763-1767, 2005.
Non-patent Document 6: Ramom-Cueto A, Cordero MI, Santos-Benito FF, Avila J: Neuron. 25: 425-435, 2000.
Non-patent Document 7: Honmou O: Spinal Surgery 30(3) 248-250, 2016
Non-patent Document 8: Kuroda Y et al. Proc Natl Acad Sci USA, 2010: 107: 8639-8643.
Non-patent Document 9: Wakao S et al. Proc Natl Acad Sci USA, 2011: 108: 9875-9880.
Non-patent Document 10: Kuroda Y et al. Nat Protc, 2013: 8: 1391-1415.

### SUMMARY OF THE INVENTION

An object of the present invention is to provide a cell product for treatment of spinal cord injury.

The present inventors have found that human Muse cells that are administered to a rat model of spinal cord injury via a blood vessel or the like, or directly to a target site of spinal cord injury and its surroundings, migrate, accumulate, and engraft to a spinal cord injury site, and reconstruct spinal cord tissues, thereby improving or restoring motor functions, and thus that the Muse cells can be used in treatment of spinal cord injury, thereby completed the present invention.

Accordingly, the present invention provides the following items [1] to [7].
[1] A cell product for treatment of spinal cord injury, comprising a SSEA-3-positive pluripotent stem cell derived from a mesenchymal tissue in a living body or a cultured mesenchymal cell.
[2] The cell product of item [1], wherein the spinal cord injury is complete spinal cord injury.
[3] The cell product of item [1], wherein the spinal cord injury is incomplete spinal cord injury.
[4] The cell product of any one of items [1] to [3], wherein said pluripotent stem cell is one having all of the following characteristics:
   (i) having low or no telomerase activity;
   (ii) capable of differentiating into any of tridermic cells;
   (iii) showing no neoplastic proliferation; and
   (iv) having self-renewal capacities.
[5] The cell product of any one of items [1] to [3], wherein said pluripotent stem cell is one having all of the following characteristics:
   (i) SSEA-3-positive;
   (ii) CD105-positive;
   (iii) having low or no telomerase activity;
   (iv) capable of differentiating into any of tridermic cells;
   (v) showing no neoplastic proliferation; and
   (vi) having self-renewal capacities.
[6] A SSEA-3-positive pluripotent stem cell derived from a mesenchymal tissue in a living body or a cultured mesenchymal cell, for use in production of a cell product for treatment of spinal cord injury.
[7] A method of treating spinal cord injury, comprising administering to a patient with spinal cord injury in need thereof a therapeutically effective amount of a cell product comprising SSEA-3-positive pluripotent stem cells derived from a mesenchymal tissue in a living body or a cultured mesenchymal cell.

According to the present invention, Muse cells can be administered to a patient with spinal cord injury via a blood vessel or the like, or directly to a target site of spinal cord injury and its surroundings, which allows the cells to migrate, accumulate, and engraft to the spinal cord injury site, and to reconstruct spinal cord tissues, thereby improving or restoring motor functions. Thus, the cell product of the present invention can be used for treatment of spinal cord injury.

Since Muse cells can efficiently migrate, accumulate, and engraft to a site of spinal cord injury, reconstructing spinal cord tissues at the engraftment site, they do not require differentiation induction into cells to be treated prior to transplantation (administration). In addition, Muse cells are non-tumorigenic and excellent in safety. Furthermore, since Muse cells do not induce any immune rejection, treatment with allogenic preparations produced from donors is also possible. Therefore, Muse cells having the excellent characteristics as described above can provide readily feasible means for treatment of patients with spinal cord injury.

### BRIEF DESCRIPTION OF THE DRAWINGS

Fig. 1 is a graph showing the results of evaluation of hindlimb motor function in a rat model of spinal cord injury treated with a vehicle (HBSS) or a Muse cell (CL2020) (Example 1-4). *P<0.05, **P<0.01 vs. HBSS
Fig. 2 is a graph showing the results of evaluation of hindlimb motor function in a rat model of spinal cord injury treated with a vehicle or a Muse cell (Example 2-1). *P<0.05, **P<0.01 vs. HBSS (Control)
Fig. 3 is a graph showing the results of evaluation of hindlimb motor function in a rat model of spinal cord injury treated with a vehicle or a Muse cell (Example 3-1). *P<0.05 vs. HBSS (Control)

### DETAILED DESCRIPTION OF THE INVENTION

The present invention relates to a cell product for treatment of spinal cord injury, comprising a SSEA-3-positive pluripotent stem cell (Muse cell). The present invention will be described in detail below.

### 1. Indications

The cell product comprising a SSEA-3-positive pluripotent stem cell (Muse cell) of the present invention is used for treatment of spinal cord injury.

As used herein, the term "spinal cord injury" refers to a condition in which the spinal cord is injured due to damage to the spine caused mainly by strong external forces applied to the spinal column. It is also known that similar disorders develop by internal causes, such as spinal cord tumors and hernias, and the cell products of the present invention can also be applied in such cases. Spinal cord injuries are divided into "complete type" and "incomplete type" according to the degree of injury. The "complete type" is referred to as "complete spinal cord injury," and is a condition in which the spinal cord is severed laterally and the neurotransmitter function is completely cut off. The "incomplete type" is referred to as "incomplete spinal cord injury," and is a condition in which a part of the spinal cord is subjected to trauma, compression, or the like with some function remaining.

Spinal cord injury presents a complex pathology that changes over time after injury, depending on the biological response to the injury. Thus, an appropriate treatment is required for each pathology at each phase.

The acute phase of spinal cord injury is classified as primary injury due to external forces, and secondary injury, a subsequent biochemical and biological response. Secondary injury collectively refers to processes of cell death of nerve and glial cells caused by hematoma, ischemia, edema, inflammatory cell infiltration, cytotoxicity due to neurotransmitter leakage, or the like, and expansion of the damage area. Secondary injury, unlike primary injury, is a condition that may be the primary target of various treatments. Therefore, treatment to reduce secondary injury and minimize tissue damage can improve the functional prognosis. The acute phase, during which the secondary injury has occurred, varies from patient to patient, for example, about three weeks after injury.

The subacute phase of spinal cord injury is the time during which inflammation in the acute phase returns to normal and angiogenic and tissue repair responses are activated. The subacute phase, during which the above-described reactions have occurred, varies from patient to patient, for example, about 1 to 12 weeks after injury.

The chronic phase of spinal cord injury results in a cavity formation in the site of spinal cord injury, leading to tissue defects. Astrocytes accumulated around the injured site form hard glial scars that act as a barrier against axonal regeneration. In addition, the chronic phase of spinal cord injury is also known to have reduced neuronal activity, which shrinks the cell body, suggesting that the response to therapy may be reduced. The chronic phase, during which the above-described conditions have occurred, varies from patient to patient, for example, about 4 weeks or later after injury.

### 2. Cell product

### (1) Pluripotent Stem Cell (Muse Cell)

The pluripotent stem cell used in the cell product of the present invention is a cell that was found in human living body and named "Muse (Multilineage-differentiating Stress Enduring) cell." It is known that Muse cells can be obtained from, for example, bone marrow aspirates, adipose tissues (Ogura, F., et al., Stem Cells Dev., Nov 20, 2013 (Epub) (published on Jan 17, 2014)) and dermal connective tissues of skin, and are broadly present in tissues and connective tissues in organs. This cell also has both characteristics of pluripotent stem cell and mesenchymal stem cell and is identified as, for example, a cell positive for "SSEA-3," a cell surface marker, preferably as a double-positive cell that is positive for SSEA-3 and CD-105. Therefore, Muse cells or a cell population containing Muse cells can be isolated from living tissues using, for example, expression of SSEA-3 only or a combination of SSEA-3 and CD-105 as an index. Methods for separation and identification of, and characteristics of Muse cells have been disclosed in WO2011/007900 in detail. Taking advantage of the high resistance of Muse cells to various external stresses, Muse cells can be selectively enriched by culturing the cells under various external stress conditions, such as under protease treatment, under hypoxic conditions, under hypophosphate conditions, in a low serum concentration, under undernutrition conditions, under heat shock exposure, in the presence of toxic substances, in the presence of active oxygen, under mechanical stimulation, and under pressure treatment. As used herein, pluripotent stem cells prepared from mesenchymal tissues in a living body or cultured mesenchymal tissues using SSEA-3 as an index (Muse cells), or a cell population comprising Muse cells, as a cell product for treating spinal cord injury, may be simply referred to as "SSEA-3-positive cells."

Muse cells or a cell population comprising Muse cells can be prepared from living tissues (e.g., mesenchymal tissues) using cell surface markers, SSEA-3, or SSEA-3 and CD-105, as an index(es). As used herein, the term "living" body means mammal living body. In the present invention, living bodies exclude fertilized egg and embryos in developmental stages before blastula stage, but include embryos in developmental stages of blastula stage or later, including fetus and blastula. Examples of the mammal include, but not limited to, primates such as human and monkey; rodents such as mouse, rat, rabbit, and guinea pig; and cat, dog, sheep, pig, cattle, horse, donkey, goat, and ferret. Muse cells to be used in the cell product of the present invention are directly isolated from living tissues using markers, and thus are clearly distinguished from embryonic stem cells (ES cells) and iPS cells. The term "mesenchymal tissue" refers to tissues such as bone, synovial membrane, fat, blood, bone marrow, skeletal muscle, dermis, ligament, tendon, dental pulp, umbilical cord, cord blood, and amnion, as well as tissues present in various organs. For example, Muse cells can be obtained from bone marrow, skin, adipose tissues, blood, dental pulp, umbilical cord, cord blood, or amnion. Preferably, a mesenchymal tissue in a living body is collected, and then Muse cells are prepared from the tissue and used. Alternatively, using the preparation method described above, Muse cells may be prepared from cultured mesenchymal cells such as fibroblasts and bone marrow mesenchymal stem cells.

The cell population comprising Muse cells to be used in the cell product of the present invention can also be prepared by a method comprising stimulating mesenchymal tissues in a living body or cultured mesenchymal cells with an external stress to selectively increase cells having the resistance to the external stress and collecting the cells with an increased abundance ratio.

The external stress may be any one of or a combination of the following: protease treatment, culturing under low oxygen concentration, culturing under hypophosphate conditions, culturing under low serum concentration, culturing undernutrition conditions, culturing under heat shock exposure, culturing at low temperatures, freezing treatment, culturing in the presence of toxic substances, culturing in the presence of active oxygen, culturing under mechanical stimulation, culturing under shaking, culturing under pressure treatment or physical shocks.

The protease treatment is preferably carried out for 0.5 to 36 hours in total to exert an external stress. The concentration of the protease may be that used when cells adhered to a culture vessel are peeled off, when cell aggregates are separated into single cells, or when single cells are collected from a tissue.

Preferably, the protease is a serine protease, an aspartic protease, a cysteine protease, a metalloprotease, a glutamic protease, or an N-terminal threonine protease. More preferably, the protease is trypsin, collagenase, or dispase.

Muse cells to be used in the cell product of the present invention may be autologous or allogeneic to a recipient who will receive the cells.

As described above, Muse cells or a cell population comprising Muse cells can be prepared from living tissues, for example, by using SSEA-3 positivity or SSEA-3 and CD-105 double positivity as an index. Human adult skin is known to comprise various types of stem cells and precursor cells. However, Muse cells are different from these cells. These stem cells and precursor cells include skin-derived precursor cells (SKP), neural crest stem cells (NCSC), melanoblasts (MB), pericytes (PC), endothelial precursor cells (EP), and adipose-derived stem cells (ADSC). Muse cells can be prepared using "non-expression" of markers unique to these cells as an index. More specifically, Muse cells can be isolated using as an index non-expression of at least one, e.g., 2, 3, 4, 5, 6, 7, 8, 9, 10, or 11, of 11 markers selected from the group consisting of CD34 (a marker for EP and ADSC), CD117 (c-kit) (a marker for MB), CD146 (a marker for PC and ADSC), CD271 (NGFR) (a marker for NCSC), NG2 (a marker for PC), vWF factor (von Willebrand factor) (a marker for EP), Sox10 (a marker for NCSC), Snail (a marker for SKP), Slug (a marker for SKP), Tyrp1 (a marker for MB), and Dct (a marker for MB). Muse cells can be prepared by using as an index non-expression of, for example, but not limited to, CD117 and CD146; CD117, CD146, NG2, CD34, vWF, and CD271; or the above-described 11 markers.

Muse cells having the above-described characteristics and used in the cell product of the present invention may also have at least one selected from the group consisting of the following characteristics:
(i) having low or no telomerase activity;
(ii) capable of differentiating into any of tridermic cells;
(iii) showing no neoplastic proliferation; and
(iv) having self-renewal capacities.
Preferably, Muse cells to be used in the cell product of the present invention has all of the characteristics described above.

With respect to (i) above, the phrase "having low or no telomerase activity" means that the telomerase activity is low or undetectable when detected using, for example, TRAPEZE XL telomerase detection kit (Millipore Corporation). Having "low" telomerase activity means, for example, having a telomerase activity comparable to somatic human fibroblast, or having 1/5 or less telomerase activity, preferably 1/10 or less telomerase activity, as compared with that of HeLa cell.

With respect to (ii) above, Muse cells are capable of differentiating into tridermic cells (endodermal, mesodermal, and ectodermal cells) in vitro and in vivo, for example, into hepatocytes (including cells expressing hepatoblastoma or hepatocyte markers), neurons, skeletal muscle cells, smooth muscle cells, osteocytes, or adipocytes by in vitro inductive culturing. Muse cells may also show the ability to differentiate into tridermic cells when transplanted in testis in vivo. Further, Muse cells are capable of migrating and engrafting to injured organs (such as heart, skin, spinal cord, liver, and muscle) when transferred to a living body via intravenous injection and differentiating into cells depending on the tissues.

With respect to (iii) above, Muse cells are characterized in that they proliferate at a growth rate of about 1.3 days and proliferate from a single cell in suspension culture to form embryoid body-like cell aggregates, and then arrest their proliferation after about 14 days when the aggregates reach a certain size. When these embryoid body-like cell aggregates are transferred to adherent culture, the cells restart proliferation and cells proliferated from the cell aggregates spread at a growth rate of about 1.3 days. Further, Muse cells are characterized in that, when transplanted into testis, they do not become cancerous for at least half a year.

With respect to (iv) above, Muse cells have self-renewal (self-replication) capacities. The term "self-renewal," as used herein, means that the followings can be observed: differentiation into tridermic cells from cells contained in first embryoid body-like cell aggregates obtained by culturing single Muse cells in a suspension culture; as well as formation of next-generation second embryoid body-like cell aggregates by again culturing single cells of the first embryoid body-like cell aggregates in a suspension culture; and further differentiation into tridermic cells and formation of third embryoid body-like cell aggregates in a suspension culture from the second embryoid body-like cell aggregates. Self renewal may be repeated for one or more cycles.

### (2) Preparation and Use of Cell product

The cell product of the present invention can be obtained by, for example, suspending Muse cells or a cell population comprising Muse cells obtained in (1) above in a physiological saline or a suitable buffer solution (e.g., a phosphate buffered saline). In this case, if only small numbers of Muse cells are isolated from an autologous or allogeneic tissue, these cells may be cultured before cell transplantation until the determined number of cells is attained. As previously reported (WO2011/007900), since Muse cells do not become tumorigenic, even if cells collected from a living tissue are contained while remaining undifferentiated, they are less likely to become cancerous and thus are safe. The collected Muse cells can be cultured in any normal growth medium (e.g., alpha-minimum essential medium (a-MEM) supplemented with 10% calf serum). More specifically, with reference to the above-described WO2011/007900, Muse cells can be cultured and proliferated using an appropriately selected culture medium, additives (e.g., antibiotics, and serum) and the like, to prepare a solution containing Muse cells at the determined concentration. When the cell product of the present invention is administered to human subject, bone marrow aspirates are collected from a human ilium. Then, for example, bone marrow mesenchymal stem cells are cultured as adherent cells obtained from the bone marrow aspirate and proliferated until reaching the cell amount where a therapeutically effective amount of Muse cells can be obtained. Thereafter, Muse cells are isolated using an antigenic marker SSEA-3 as an index to obtain a cell product containing autologous or allogeneic Muse cells. Alternatively, for example, bone marrow mesenchymal stem cells obtained from the bone marrow aspirates can be cultured under external stress conditions, so that Muse cells can be grown and enriched until they reach a therapeutically effective amount, to obtain a cell product comprising autologous or allogeneic Muse cells.

When Muse cells are used in a cell product, the cell product may also comprise dimethyl sulfoxide (DMSO), serum albumin and the like for protection of the cells and antibiotics and the like for prevention of contamination and proliferation of bacteria. The cell product may further comprise other pharmaceutically acceptable components (e.g., carriers, excipients, disintegrants, buffer agents, emulsifiers, suspending agents, soothing agents, stabilizers, preservatives, antiseptics, physiological saline). These agents and drugs can be added to a cell product at appropriate concentrations by the skilled person. Thus, Muse cells can also be used as a pharmaceutical composition comprising various additives. The dosage form of the cell product is not particularly restricted, and is preferably a parenteral formulation, more preferably an injectable formulation.

The number of Muse cells contained in the cell product prepared above can be appropriately adjusted to achieve desired effects in treatment of spinal cord injury, in consideration of, for example, sex, age, and weight of the subject, condition of the affected area, and the condition of the cells to be used. Individuals as the subject includes, but not limited to, mammals such as human. The cell product of the present invention may be administered in a single dose, or may be administered multiple times (e.g., 2 to 10 times) at appropriate intervals (e.g., twice a day, once a day, twice a week, once a week, once every two weeks, once a month, once every two months, once every three months, or once every six months) until the desired therapeutic effect is obtained. The timing of administration may be either in the acute, subacute, or chronic phase as long as the therapeutic effect can be obtained. Alternatively, two or more of these periods may be selected.

Preferably, the therapeutically effective amount is, for example, 1 to 10 doses of 1×10³ to 1×10¹⁰ cells/individual/dose, depending on the state of the subject. The total amount administered to an individual is, for example, 1×10³ to 1×10¹¹ cells, preferably 1×10⁴ to 1×10¹⁰ cells, more preferably 1×10⁵ to 1×10⁹ cells.

Muse cells to be used in the cell product of the present invention is characterized in that they migrate and engraft to a damaged site of spinal cord injury. Thus, the cell product may be administered to any site by any method, e.g., locally to the affected area or intravenously.

The cell product of the present invention can reconstruct the spinal cord tissue in a patient with spinal cord injury and improve or recover the lost functions due to the spinal cord injury.

In general, an animal model is used to measure the effect of the cell product of the present invention for treatment of spinal cord injury. Animal species to be used include, in general, rats and mice, and may also include monkeys, dogs, and rabbits. The spinal cord of these animals is subjected to direct injury, e.g., amputation, crush by impact, or electrical or thermal injury, to create the pathology of spinal cord injury.

For evaluation of the extent of injury and recovery with treatment, evaluations based on, for example, limb movement, body position, or limb sensation, such as Basso-Beattie-Bresnahan (BBB) score, tactile stimulation (VFH study: Experimental Neurology 225, Issue 2, 366-376, (2010)), or Catwalk gait analysis (Abhiraj D. Bhiman et al., Neuroscience & Biobehavioral Reviews 83; 540-546 (2017)), as well as histopathological evaluations are used.

In clinical practice, the Frankel's Classification or American Spinal Injury Association Impairment (ASIA) scale is often used (Committee for the Preparation of Guidelines for the Treatment and Management of Spinal Cord Injury: Guidelines for the Treatment and Management of Spinal Cord Injury, Spinal Surgery 2005; 19: (supple 1): 1-41).

The present invention will be described in more detail with reference to examples below, but is not limited to the examples.

### EXAMPLES

### Example 1-1: Preparation of Rat Model of Spinal Cord Injury

An 11-week-old female SD rat was anesthetized, then subjected to laminectomy of the 9-10th thoracic vertebrae to expose the spinal cord, and then a weight (2.5 mm in diameter, 10 g in weight) was immediately dropped from a height of 25 mm using a MASCIS Impactor (Rutgers University, USA) to injure the spinal cord (8 rats each in 3 groups).

### Example 1-2: Preparation of Human Muse Cell

Muse cells were obtained according to the method described in WO2011/007900 on isolation and identification of human Muse cells. More specifically, Muse cells were obtained by expansive enrichment culture of mesenchymal stem cells under stress conditions.

### Example 1-3: Administration of Cells to Rat Model of Spinal Cord Injury

Rats were divided into three groups of eight rats each. The first group received 5 × 10⁶ cells/kg of body weight of Muse cells by tail vein injection at Day 1 after spinal cord injury (single dose group). The second group received each 5 × 10⁶ cells/kg of body weight of Muse cells by tail vein injection at Days 1 and 7 after spinal cord injury (multiple dose group). The third group received a vehicle (HBSS) by tail vein injection at Day 1 after spinal cord injury (control group).

### Example 1-4: Evaluation of Hindlimb Motor Function

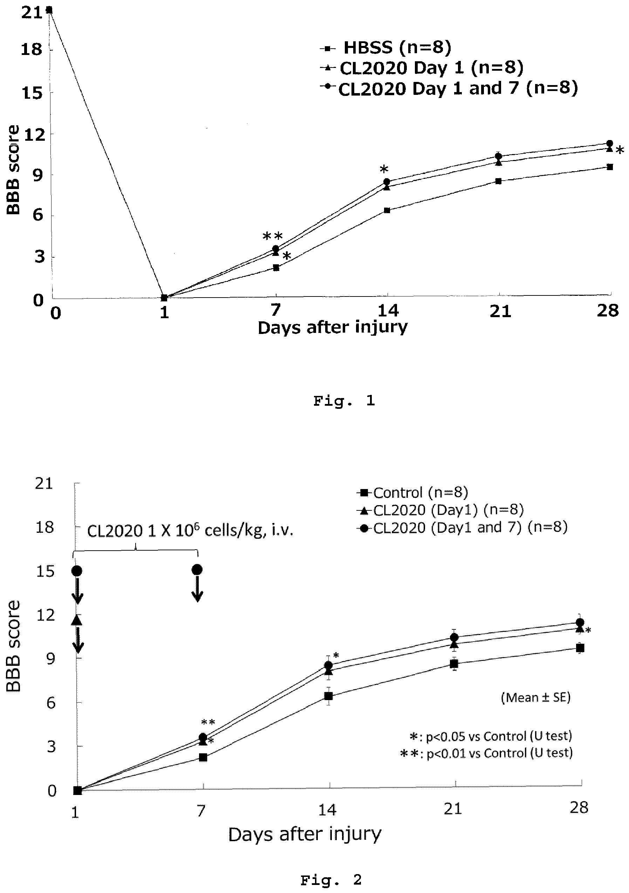

For the rats in each group, the hindlimb motor function was examined based on the Basso-Beattie-Bresnahan (BBB) score (J. Neurotrauma 1995; 12: 1-21) once a week until 4 weeks after spinal cord injury. As shown in the results in Fig. 1, the Muse cell-treated groups consistently showed higher BBB scores. At Day 7 or later after spinal cord injury, the Muse-cell single-dose and multiple-dose groups showed significantly higher scores as compared to the vehicle-treated group, indicating an improving effect on the hindlimb motor function by Muse cells.

### Example 2-1: Administration (in acute phase) of Muse Cells to Rat Model of Spinal Cord Injury and Evaluation of Hindlimb Motor Function

In the same manner as in Examples 1-1 to 1-4, Muse cells were administered to a rat model of spinal cord injury, and the hindlimb motor function was evaluated. However, the dose of Muse cells was 1×10⁶ cells/kg of body weight. The BBB score results are shown in Fig. 2. The results showed that Muse cells, when administered in the acute phase of spinal cord injury, showed significantly superior improvement or recovery effects on the motor function than the level of spontaneous improvement or recovery observed immediately after spinal cord injury in the control group.

### Example 2-2: Histopathological Analysis in Muse Cell-treated (in Acute Phase) Rat Model of Spinal Cord Injury

The Muse cell-treated groups and the control group in Example 2-1 were subjected to the last BBB score measurement at Day 28 after spinal cord injury, followed by isolation of and histopathological analysis on the spinal cord. Specifically, the isolated spinal cord was fixed with formalin, prepared into a paraffin section according to the conventional method, stained with hematoxylin and eosin (HE) staining as well as by Klüver-Barrera (KB) staining, and observed by optical microscopy. The spinal cord was cut out in coronal cross-sections at the center of the injured area, and at 5 mm each of its rostral and caudal sides to obtain surfaces for observation. Observation included all histopathological changes. In the case that any findings different from normal were observed, they were classified depending on the extent of the findings, as grade 1 for minor changes, grade 2 for moderate changes, and grade 3 for severe changes. For example, in the case of cavitation, grade 3 was defined as when the changes accounted for 50% or more of the spinal cross-section, grade 2 was defined as when the changes accounted for 10 to 50%, and grade 1 was defined as when the changes accounted for 10% or less.

The results revealed that both the control and Muse cell-treated groups showed remarkable emergence of foamy macrophages, growth of glial cells, and/or swelling of axon, and some showed cavitation, demyelination, and/or hemosiderosis. Observation at each evaluation site of spinal cord revealed that changes in the emergence of foamy macrophages and growth of glial cells in the injured site tended to be slightly higher in grade in the Muse cell-treated group than in the control group, while the changes in the caudal side of the injured site tended to be slightly lower in grade in the Muse cell-treated group than in the control group.

### Example 3-1: Administration (in subacute to chronic phase) of Muse Cells to Rat Model of Spinal Cord Injury and Evaluation of Hindlimb Motor Function

In the same manner as in Examples 1-1 to 1-4, Muse cells were administered to a rat model of spinal cord injury, and the hindlimb motor function was evaluated. However, Muse cells (1×10⁶ cells/kg of body weight) were administered once at Day 14 after spinal cord injury for the first group (single dose group), and once each at Days 14 and 28 after spinal cord injury for the second group (multiple dose group), while a vehicle (HBSS) was administered at Day 14 after spinal cord injury for the third group (control group).

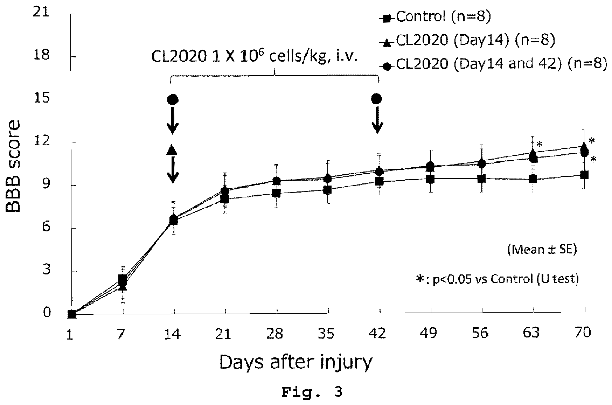

The BBB score results are shown in Fig. 3. The results showed that Muse cells, when administered in the subacute to chronic phase of spinal cord injury, resulted in further improvement or recovery of the motor function even in the steady-state phase, during which spontaneous improvement or recovery of the motor function after spinal cord injury usually reaches a ceiling. The improvement or recovery effect on the motor function was continued for 70 days or longer after spinal cord injury.

### Example 3-2: Histopathological Analysis in Muse Cell-treated (in Subacute to Chronic Phase) Rat Model of Spinal Cord Injury

The Muse cell-treated groups and the control group in Example 3-1 were subjected to the last BBB score measurement at Day 70 after spinal cord injury, followed by isolation of and histopathological analysis on the spinal cord. Specifically, the isolated spinal cord was fixed with formalin, prepared into a paraffin section according to the conventional method, stained with hematoxylin and eosin (HE) staining as well as by Klüver-Barrera (KB) staining, and observed by optical microscopy. The spinal cord was cut out in coronal cross-sections at the center of the injured area, and at 5 mm each of its rostral and caudal sides to obtain surfaces for observation. In addition to Example 2-2, the number of regenerated myelin sheaths was also measured. The number of regenerated myelin sheaths was calculated as the ratio of the number of cells having a regenerated myelin sheath to the number of cell nuclei in a given visual field.

Observation at each evaluation site of spinal cord revealed that, in particular, the number of regenerated myelin sheaths in the injured site showed an incremental tendency of 0.3409 ± 0.1836 in the Muse-cell multi-treatment group as compared to 0.2424 ± 0.2328 in the control group.

Therefore, it was suggested that administration of Muse cells had an effect to promote neural regeneration in spinal cord injury.

### Industrial Availability

The cell product of the present invention can be administered to a patient with spinal cord injury to induce reconstitution at tissues damaged with spinal cord injury and improve or recover the functions, and thus can be applied to treatment of spinal cord injury.

## Claims

1. A cell product for treatment of spinal cord injury, comprising a SSEA-3 (Stage-Specific Embryonic Antigen-3)-positive pluripotent stem cell derived from a mesenchymal tissue in a living body or a cultured mesenchymal cell.

2. The cell product according to claim 1, wherein the spinal cord injury is complete spinal cord injury.

3. The cell product according to claim 1, wherein the spinal cord injury is incomplete spinal cord injury.

4. The cell product according to any one of claims 1 to 3, wherein said pluripotent stem cell is one having all of the following characteristics:
(i) having low or no telomerase activity;
(ii) capable of differentiating into any of tridermic cells;
(iii) showing no neoplastic proliferation; and
(iv) having self-renewal capacities.

5. The cell product according to any one of claims 1 to 3, wherein said pluripotent stem cell is one having all of the following characteristics:
(i) SSEA-3-positive;
(ii) CD105-positive;
(iii) having low or no telomerase activity;
(iv) capable of differentiating into any of tridermic cells;
(v) showing no neoplastic proliferation; and
(vi) having self-renewal capacities.
